# EUROPÄISCHE PATENTSCHRIFT

(11) **EP 2 606 918 B1**
(45) Veröffentlichungstag und Bekanntmachung des Hinweises auf die Patenterteilung: **25.06.2014**
(21) Anmeldenummer: 11010131.8
(22) Anmeldetag: 21.12.2011
(51) Int. Cl.: A61M 1/06, A61M 1/00

(54) **Elektrische Muttermilchpumpe**
Electric breast milk pump
Tire-lait maternel électrique

(43) Veröffentlichungstag der Anmeldung: 26.06.2013
(73) Patentinhaber: MAPA GmbH, 27404 Zeven (DE)
(72) Erfinder: Jäger-Waldau, Reinhold, Dr., 27383 Scheessel (DE)
(74) Vertreter: Hauck Patent- und Rechtsanwälte

(56) Entgegenhaltungen:
- DE-A1-102010 019 041
- DE-B3-102004 030 692
- DE-U1- 20 220 598
- US-A- 5 542 921
- US-A1- 2008 275 386

## Beschreibung

Die Erfindung bezieht sich auf eine elektrische Muttermilchpumpe.

Muttermilchpumpen dienen dem Abpumpen von Muttermilch. Hierzu weisen sie mindestens eine Saugglocke auf, die auf die Mutterbrust aufgesetzt wird. An die Saugglocke wird ein Unterdruck angelegt, um die Milch aus der Mutterbrust abzuziehen. Dieser Unterdruck wird nachfolgend auch als "Saugunterdruck" bezeichnet. Die Saugglocke ist mit einem Behälter verbunden, der die abgesaugte Muttermilch aufnimmt. Der Unterdruck wird mittels einer Pumpe erzeugt, die manuell oder mittels eines Elektromotors angetrieben ist. Bekannt sind mittels eines Elektromotors angetriebene elektrische Muttermilchpumpen, die eine elektronische Steuerung aufweisen, die bestimmte Pumpsequenzen steuert. In diesen Pumpsequenzen wird die Pumpe während definierter Zeiträume ein- und ausgeschaltet, um den Milchfluss zu stimulieren. Die Höhe des Unterdruckes ist einstellbar.

Aus der WO 2011/137994 A ist eine elektrische Muttermilchpumpe bekannt, die eine von einem Elektromotor angetriebene Pumpe aufweist, die über eine Saugleitung mit einer Schwimmerventilkammer verbunden ist, welche über einen Verbindungskanal mit einer Saugglocke verbunden ist. Am unteren Ende des Verbindungskanals ist ein Milchablassventil in Form eines Entenschnabelventils mit einem Ventilschlitz am unteren Ende angeordnet. Saugglocke, Verbindungskanal und Unterdruckkammer sind an einem Schraubring angeordnet, der mit einem belüfteten Behälter zum Sammeln von Milch verschraubbar ist, so dass das Entenschnabelventil in die Milchflasche hineinragt. In der Schwimmerventilkammer ist ein Schwimmerkörper angeordnet, das aufschwimmt, wenn sich eine übermäßige Menge Milch in der Kammer angesammelt hat. Der aufgeschwommene Schwimmerkörper schließt die Verbindung zur Pumpe, damit die Milch nicht in die Pumpe hineingelangt und diese verstopft bzw. verklebt. Die Saugleitung ist über eine Belüftungsleitung mit der Umgebung (Atmosphäre) verbunden, in der ein elektrisch betätigbares Belüftungsventil angeordnet ist. Eine elektronische Steuerung steuert den Pumpbetrieb. Wenn die Pumpe läuft, schließt die Steuerung das Belüftungsventil, so dass in der Unterdruckkammer ein Unterdruck aufgebaut und Milch abgesaugt wird. Die abgesaugte Milch strömt durch den Verbindungskanal zum Entenschnabelventil. Das Entenschnabelventil sorgt dafür, dass der Unterdruck in der Saugglocke erhalten bleibt.

Zum Stimulieren des Milchflusses wird die Pumpe von der Steuerung zyklisch ein- und ausgeschaltet. In den Ausschaltphasen wird das Belüftungsventil geöffnet, damit der Unterdruck in der Saugglocke abgebaut wird. Durch diese Druckschwankungen wird der Milchfluss angeregt. Beispielsweise wird die Pumpe 30 mal pro Sekunde ein- und ausgeschaltet. Die Milch sammelt sich über dem Entenschnabelventil und tropft durch den Schlitz in den Behälter hinein, wenn das Belüftungsventil geöffnet wird.

Es hat sich gezeigt, dass trotz des Schwimmerventils Milch von der Pumpe angesaugt werden kann. Dies ist insbesondere bei kurzen Belüftungsphasen bzw. hohen Milchflüssen der Fall. Wenn die Pumpe Milch ansaugt, können die Ventile bzw. die Membran einer Membranpumpe verkleben, was zu einer geringeren Saugleistung bzw. zum Ausfall der Pumpe führen kann.

Die EP 1 221 319 B1 beschreibt eine Brustpumpe mit einer Membran-Vakuumpumpe mit Antriebsmotor und einer Steuerelektronik, um in Abhängigkeit eines vorprogrammierten Vakuumverlaufs die Motordrehzahl einzustellen. Zusätzlich ist eine Vakuumkammer mit Trennmembran vorgesehen, deren erste durch die Trennmembrane abgetrennte Kammer mit der Saugleitung der Vakuumpumpe in Verbindung steht und deren zweite durch die Trennmembrane abgetrennte Kammer mit einer Absaughaube verbindbar ist. Die Trennmembrane verhindert, dass Milch in die Vakuumpumpe eintritt.

Bei dieser Brustpumpe wird gemäß einem Programm ein Unterdruck an die Brust angelegt, um die Milchleistung zu stimulieren. Insbesondere Höhe und Frequenz des Unterdruckes sind vom Programm gesteuert. Ein besonders vorteilhaftes Absaugprogramm besteht bei jedem Zyklus im progressiven Aufbau des gewünschten Vakuums und Halten des maximalen Vakuums während einer vorbestimmten Zeit und anschließend einem möglichst raschen vollständigen Abbau des Vakuums auf Null-Vakuum, um nach einer Ruhepause den folgenden Zyklus auszuführen. Deshalb ist die Brustpumpe mit einer gesteuerten Einrichtung zur Aufhebung des aufgebauten Vakuums ausgerüstet. Die Einrichtung zur Aufhebung des Vakuums besteht aus einem von der Steuerelektronik gesteuerten Ventil, welches bei Öffnung die erste Kammer mit der Umgebung verbindet, d.h. die Vakuumkammer passiv entlüftet. Alternativ besteht die Einrichtung zur Aufhebung des Vakuums in der Vakuumkammer aus einer Ventilweiche, welche bei Betätigung die Saugleitung zwischen Vakuumpumpe und der ersten Kammer unterbricht und dafür die erste Kammer mit einer einen positiven Druck aufbauenden Ausstoßleitung der Vakuumpumpe verbindet, d.h. das Vakuum in der Vakuumkammer durch positiven Druckaufbau aktiv aufhebt.

Davon ausgehend liegt der Erfindung die Aufgabe zugrunde, eine elektrische Muttermilchpumpe zu schaffen, die besser vor einem Ansaugen von Milch durch die Pumpe geschützt ist.

Die Aufgabe wird durch eine elektrische Muttermilchpumpe mit den Merkmalen von Anspruch 1 gelöst. Vorteilhafte Ausgestaltungen der Muttermilchpumpe sind in Unteransprüchen angegeben.

Die erfindungsgemäße elektrische Muttermilchpumpe hat
- einen Elektromotor,
- eine von dem Elektromotor angetriebene Pumpe,
- mindestens eine Saugglocke,
- einen über einen Verbindungskanal mit der Saugglocke verbundenen Ablauf für Muttermilch,
- einen mit dem Ablauf verbundenen Behälter zum Auffangen von Muttermilch,
- ein im Ablauf angeordnetes Ablaufventil, das schließt, wenn die Differenz des Druckes im Behälter und in der Saugglocke einen bestimmten Mindestwert aufweist und das öffnet, wenn der Mindestwert unterschritten ist,
- eine Schwimmerventilkammer, die unten mit dem Verbindungskanal verbunden ist und oben eine Durchgangsöffnung sowie einen die Durchgangsöffnung umgebenden Dichtsitz hat,
- einen in der Schwimmerventilkammer angeordneten Schwimmerkörper, der oben einen Dichtkörper aufweist, der abdichtend am Dichtsitz anliegt, wenn der Schwimmerkörper in der Schwimmerventilkammer aufschwimmt,
- eine die Durchgangsöffnung mit dem Eingang der Pumpe verbindende Saugleitung,
- ein in der Saugleitung angeordnetes, elektrisch betätigbares erstes Dreiwegeventil,
- eine mit dem Verbindungskanal und dem Ausgang der Pumpe verbundene Druckleitung,
- ein in der Druckleitung angeordnetes, elektrisch betätigbares zweites Dreiwegeventil,
- Mittel zum Ein- und Ausschalten des Elektromotors und
- eine elektronische Steuerung, die mit den Mitteln zum Ein- und Ausschalten, dem Elektromotor, und den elektrisch betätigbaren ersten und zweiten Dreiwegeventilen elektrisch verbunden ist und die ausgebildet ist, den Elektromotor in Abhängigkeit von einer Betätigung der Mittel zum Ein- und Ausschalten in einen Betriebszustand und in einen Ruhezustand versetzen, wobei sie im Betriebszustand den Elektromotor und die elektrisch betätigbaren ersten und zweiten Dreiwegeventile in Zyklen betreibt, die eine Saugphase umfassen, in der der Elektromotor eingeschaltet ist, der Eingang der Pumpe über die Saugleitung und das erste Dreiwegeventil mit der Durchgangsöffnung verbunden ist und der Ausgang der Pumpe über die Druckleitung und das zweite Dreiwegeventil mit der Umgebung verbunden ist, die ferner eine Druckphase umfassen, in denen der Elektromotor eingeschaltet ist, der Eingang der Pumpe über die Saugleitung und das erste Dreiwegeventil mit der Umgebung verbunden ist und der Ausgang der Pumpe über die Druckleitung und das zweite Dreiwegeventil mit dem Verbindungskanal verbunden ist.

Der Erfindung liegt die Erkenntnis zugrunde, dass bei der bekannten Muttermilchpumpe mit elektronisch gesteuertem Belüftungsventil in den Belüftungsphasen der Druckausgleich in der Saugglocke bei kurzen Zyklen bzw. hohen Milchflüssen nicht schnell genug stattfindet, so dass die abgepumpte Milch nicht oder nicht hinreichend durch das Ablaufventil in den Behälter abläuft. Infolgedessen kann es dazu kommen, dass sich die Milch oberhalb des Ablaufventils allmählich anstaut, bis zum Dichtsitz des Schwimmerventiles aufsteigt und in die Pumpe hineingelangt. Dies kann insbesondere bei kleinen Muttermilchpumpen geschehen, bei denen die Belüftungsleitung und das Belüftungsventil kleine Abmessungen haben, wodurch der Durchfluss beim Druckausgleich begrenzt wird. Erfindungsgemäß wird dieses Problem überwunden, indem im Betriebszustand in den Druckphasen mittels der Pumpe aktiv Luft in den Verbindungskanal und damit in die Saugglocke und in den Ablauf gepumpt wird, so dass der Druck über dem Ablaufventil schneller als bei passiver Belüftung auf Umgebungsdruck oder sogar auf einen kleinen Überdruck gegenüber dem Umgebungsdruck gebracht werden kann. Durch den angestiegenen Druck über dem Ablaufventil öffnet das Ablaufventil. Infolgedessen kann die in den Saugphasen abgepumpte und am Ablaufventil anstehende Milch zwischen den Saugphasen hinreichend durch das Ablaufventil in den Behälter abfließen und steigt nicht zum Ventilsitz des Schwimmerventils auf. Die Milch fließt besonders schnell ab, wenn in dem Verbindungskanal ein kleiner Überdruck gegenüber Umgebungsdruck eingestellt wird. Die Zeitspanne für das aktive Einpumpen von Luft in den Verbindungskanal kann kürzer sein als die Spanne zwischen zwei aufeinanderfolgenden Saugphasen. In der verbleibenden Zeit zwischen zwei Saugphasen kann der Elektromotor abgeschaltet werden, um Energie einzusparen und den Motorverschleiß zu mindern. Im Ruhezustand ist der Elektromotor ebenfalls abgeschaltet.

Gemäß einer Ausgestaltung ist die elektronische Steuerung so ausgebildet, dass in einer Belüftungsphase im Anschluss an die Druckphase oder zwischen der Saugphase und der Druckphase der Elektromotor ausgeschaltet ist, der Eingang der Pumpe über die Saugleitung und das erste Dreiwegeventil mit der Umgebung (Atmosphäre) verbunden ist und der Ausgang der Pumpe über die Druckleitung und das zweite Dreiwegeventil mit dem Verbindungskanal verbunden ist. In der Belüftungsphase erfolgt durch passive Belüftung der Saugglocke durch die Pumpe hindurch ein teilweiser oder vollständiger Druckausgleich mit der Umgebung. Die Belüftungsphase kann der Druckphase nachfolgen, so dass nach der Saugphase zunächst die angesammelte Milch in den Behälter sicher abgegeben wird. Stattdessen kann die Belüftungsphase der Saugphase direkt nachfolgen und durch eine an die Belüftungsphase anschließende Druckphase sichergestellt werden, dass die angesammelte Milch in den Behälter abfließt. Wenn die Belüftungsphase an die Druckphase anschließt, kann beim Übergang von der Saugphase zur Druckphase der Elektromotor weiterlaufen und müssen nur die Dreiwegeventile umgeschaltet werden. Wenn die Belüftungsphase an die Saugphase anschließt, kann beim Übergang von der Druckphase in die nächste Saugphase der Elektromotor weiterlaufen, wobei die Dreiwegeventile umgeschaltet werden müssen.

Gemäß einer anderen Ausgestaltung ist die elektronische Steuerung so ausgebildet, dass sie in einer Belüftungsphase zwischen Saugphase und Druckphase den Eingang der Pumpe über die Saugleitung und das erste Dreiwegeventil und den Ausgang der Pumpe über die Druckleitung und das zweite Dreiwegeventil mit der Umgebung verbindet. Bei dieser Ausgestaltung erfolgt durch zwangsläufig vorhandene Leckagen in der Belüftungsphase ein allmählicher Druckausgleich mit der Umgebung.

Gemäß einer Ausgestaltung sind Mittel zum Einstellen der Drehzahl des Elektromotors vorhanden und ist die elektronische Steuerung so ausgebildet, dass sie den Elektromotor mit einer Drehzahl entsprechend der Einstellung der Mittel zum Einstellen der Drehzahl laufen lässt. Hierdurch ist der von der Pumpe erzeugte Saugunterdruck den Anforderungen entsprechend einstellbar. Im Vergleich zu Muttermilchpumpen, bei denen mittels einer permanenten, regulierbaren Belüftung der Saugunterdruck einstellbar ist, wird weniger elektrische Energie benötigt und der Verschleiß des Elektromotors verringert. Gemäß einer weiteren Ausgestaltung ist die elektronische Steuerung so ausgebildet, dass sie entsprechend der Einstellung der Mittel zum Einstellen den Elektromotor kontinuierlich oder in Zyklen betreibt. Je nach Bedarf der Anwenderin kann die Pumpe ununterbrochen arbeiten oder in Zyklen mit Saug- und Druckphasen und gegebenenfalls Belüftungsphasen.

Gemäß einer weiteren Ausgestaltung ist das erste Dreiwegeventil und/oder das zweite Dreiwegeventil ein Drei/Zwei-Wegeventil. Hierbei handelt es sich um ein spezielles Dreiwegeventil, das zwei Ventilstellungen aufweist. Grundsätzlich kann das Dreiwegeventil auch mehr als zwei Ventilstellungen aufweisen. Ferner kann als Dreiwegeventil ein Ventil eingesetzt werden, bei dem mehr als zwei Durchflusswege geöffnet und geschlossen werden können, von denen im Rahmen der Erfindung nur zwei öffen- und schließbare Durchflusswege genutzt werden.

Gemäß einer weiteren Ausgestaltung ist das erste Dreiwegeventil und/oder das zweite Dreiwegeventil ein elektromagnetisches Ventil. Weiterhin bevorzugt ist das elektromagnetische Ventil ein Solenoidventil. Es ist aber auch der Einsatz anderer elektrisch betätigbarer Ventile möglich, beispielsweise ein mittels eines Piezoantriebs betätigbares Ventil.

Gemäß einer weiteren Ausgestaltung ist die Saugglocke über ein Überdruckventil mit der Umgebung verbunden. Das Überdruckventil ermöglicht einen Druckausgleich mit dem Umgebungsdruck, wenn in der Saugglocke ein überhöhter Druck ansteht.

Es versteht sich, dass die Muttermilchpumpe eine Stromversorgung aufweist, die den Elektromotor, das erste und zweite Dreiwegeventil und die elektronische Steuerung speist. Hierbei kann es sich insbesondere um eine Batterie, einen Akkumulator, ein Ladegerät bzw. ein Netzteil handeln. Kombinationen der genannten Stromversorgungen sind möglich.

Gemäß einer Ausgestaltung ist der Elektromotor ein Gleichstrommotor mit Schleifer und Kommutator. Derartige Motoren, die für eine Betriebsdauer von einigen 100 Stunden konzipiert sind, stehen in kompakter Bauweise und geeigneter Leistungsstärke zur Verfügung.

Gemäß einer Ausgestaltung weist die elektronische Steuerung eine Platine mit einem Mikrorechner auf, auf dem ein oder mehrere Programme zur Steuerung des Elektromotors und des ersten sowie des zweiten Dreiwegeventils gespeichert sind. Verschiedene Programme können durch wahlweise Anbringung von Steckbrücken an verschiedenen Positionen der Platine auswählbar sein. Die Steckbrücken verbinden Leiterbahnen der Platine, die mit dem Mikrorechner verbunden sind. Je nachdem, welche Leiterbahnen verbunden sind, wird der Mikrorechner in verschiedenen Schaltzuständen betrieben, in denen der Mikrorechner verschiedene Programme ausführt. Durch Anbringung der Steckbrücken ist es bei der Produktion möglich, gewünschte Programme der Pumpe auszuwählen. Hierdurch kann eine Familie von Muttermilchpumpen mit verschiedenen Pumpsequenzen zur Verfügung gestellt werden. Hierfür ist lediglich die unterschiedliche Anbringung von Steckbrücken erforderlich. Die Steckbrücken können beispielsweise in die Platine eingelötete Drahtbrücken sein.

Gemäß einer weiteren Ausgestaltung ist die Pumpe eine Membranpumpe. Mittels einer Membranpumpe können bei kompakter Bauweise die erforderlichen Saugunterdrucke in kurzer Zeit aufgebaut werden. Die Saugunterdrucke liegen bevorzugt im Bereich von 330 bis 50 mbar. In den Saugphasen können diese Saugunterdrucke mittels einer Membranpumpe in einer Zeit im Sekundenbereich oder darunter aufgebaut werden. Anstatt einer Membranpumpe kann insbesondere eine Kolbenpumpe oder eine Schlauchpumpe zum Einsatz kommen.

Gemäß einer weiteren Ausgestaltung ist das Ablaufventil ein Entenschnabelventil. Ein Entenschnabelventil ist ein im Querschnitt V-förmiges Ventil mit einem Schlitz an der Kontaktlinie zwischen den Schenkeln des V. Das Entenschnabelventil schließt, wenn die Differenz der unterhalb und oberhalb der Schenkel anstehenden Drucke einen bestimmten Mindestwert aufweist und öffnet, wenn der Mindestwert unterschritten wird. Der Druck oberhalb des Ablaufventils wird durch die Menge der anstehenden Flüssigkeit und den durch die Pumpe angelegten Saugunterdruck oder Umgebungsdruck oder Überdruck bestimmt. Der Druck unterhalb des Ablaufventils ist bei Belüftung des Behälters der Umgebungsdruck. Statt eines Entenschnabelventils, können auch beliebig andere mechanische Rückschlagventile verwendet werden.

Gemäß einer weiteren Ausgestaltung weist die Muttermilchpumpe einen lösbar mit dem Behälter verbundenen Deckel auf, an dem die Saugglocke, der Verbindungskanal, die Schwimmerventilkammer und der Ablauf mit dem Ablaufventil angeordnet sind. Der Deckel ist bevorzugt ein Schraubdeckel mit einem Schraubgewinde, das mit einem komplementären Gewinde am Rand des Behälters verschraubbar ist.

Bevorzugt weist der Deckel einen Belüftungskanal zum Belüften des Behälters auf, an dem der Deckel fixiert ist.

Gemäß einer weiteren Ausgestaltung sind die Saugglocke, der Verbindungskanal, der Ablauf und das Schwimmergehäuse einteilig mit dem Deckel verbunden.

Gemäß einer weiteren Ausgestaltung sind der Elektromotor, die Pumpe, die elektronische Steuerung, das zweite Dreiwegeventil und eine Stromversorgung in einem Gehäuse zusammengefasst.

Nachfolgend wird die Erfindung anhand der anliegenden Zeichnungen von Ausführungsbeispielen der Erfindung näher erläutert. In den Zeichnungen zeigen:
- Fig. 1a und b: eine erfindungsgemäße Muttermilchpumpe in einem grobschematischen Vertikalschnitt mit zugehöriger pneumatischer und elektronischer Schaltung in der Saugphase (Fig.la) und in der Druckphase (Fig. 1b);
- Fig. 2: den Verlauf des Druckes über der Zeit bei einer Betriebsweise der Muttermilchpumpe, bei der sich jeweils an die Saugphase eine Druckphase und daran eine Belüftungsphase anschließt;
- Fig. 3: Verlauf des Druckes über der Zeit einer weiteren Betriebsweise der Muttermilchpumpe, bei der sich jeweils an die Saugphase eine Belüftungsphase und an die Belüftungsphase eine Druckphase anschließt;
- Fig. 4a bis c: ein pneumatisches Schaltbild einer Muttermilchpumpe mit Stellung der Zwei/Drei-Wegeventile in der Saugphase (Fig. 4a), in der Belüftungsphase mit Druckausgleich durch Leckagen (Fig. 4b) und in der Druckphase (Fig. 4c);
- Fig. 5: Verlauf des Druckes über der Zeit bei einer Betriebsweise, bei der jeweils auf eine Saugphase eine Belüftungsphase mit Druckausgleich durch Leckagen und auf die Belüftungsphase eine Druckphase folgt;
- Fig. 6a bis 13a und 6b bis 13b: Varianten des pneumatischen Schaltbildes der Muttermilchpumpe mit der Stellung der Drei/Zwei-Wegeventile in der Saugphase (Fig. 6a bis 13a) und mit der Stellung der Drei/Zwei-Wegeventile in der Druckphase (Fig. 6b bis 13b).

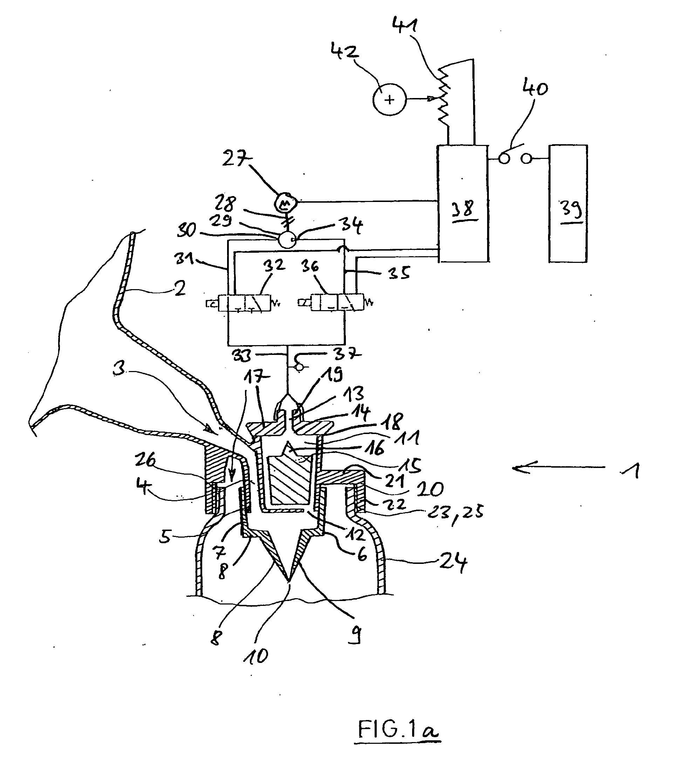

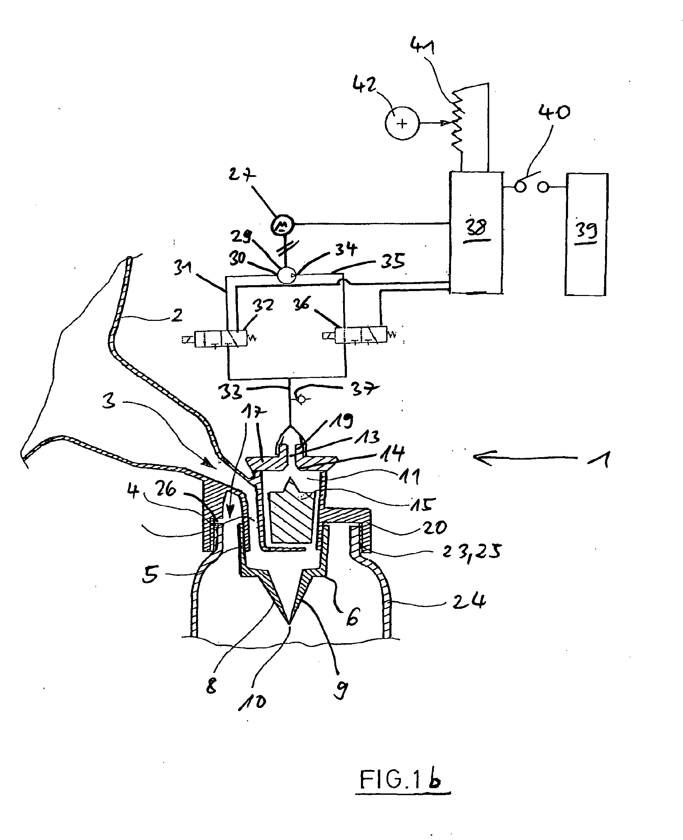

Gemäß Fig. 1a und b weist eine Muttermilchpumpe 1 eine Saugglocke 2 auf, die sich nach außen erweitert und an ihrem inneren Ende eine Öffnung 3 zu einem Verbindungskanal 4 aufweist. Der Verbindungskanal 4 geht am unteren Ende in einen Ablauf 5 in Form eines vertikal gerichteten Rohrstutzens über.

Auf dem Ablauf 5 sitzt ein Ablassventil 6 in Form eines Entenschnabelventils. Das Entenschnabelventil 6 ist aus einem elastischen und inerten Material hergestellt, beispielsweise aus Silikonkautschuk oder aus Latex. Das Entenschnabelventil 6 weist oben einen rohrförmigen Abschnitt 7 auf, der unten einen Ventilboden 8 aufweist, von dem zwei flache Schenkel 8, 9 nach unten vorstehen. Das Entenschnabelventil 6 ist mit dem rohrförmigen Abschnitt auf den Rohrstutzen aufgeklemmt. Die Schenkel 8, 9 sind aufeinander zu geneigt und zwischen ihren unteren Enden ist ein Ventilschlitz 10 vorhanden.

Ferner weist die Muttermilchpumpe 1 eine zylindrische oder konische Schwimmerventilkammer 11 auf, die vertikal ausgerichtet ist. Die Schwimmerventilkammer 11 ist unten über eine Verbindungsöffnung 12 mit dem Verbindungskanal 4 verbunden. Oben hat die Schwimmerventilkammer 11 eine Durchgangsöffnung 13, um die herum ein konischer Dichtsitz 14 vorhanden ist.

In der Schwimmerventilkammer 11 ist ein Schwimmerkörper 15 angeordnet, dessen äußere Form an den Innenraum der Schwimmerventilkammer 11 angepasst ist, so dass er in der Schwimmerventilkammer 11 aufschwimmen kann. Der Schwimmerkörper 11 trägt oben zentral einen konischen Dichtkörper 16, der eine zum Dichtsitz 14 komplementäre Form aufweist.

Die Durchgangsöffnung 13 ist in einem Kammerdeckel 17 der Schwimmerventilkammer 11 ausgebildet, der nach Einsetzen des Schwimmerkörpers 15 lösbar oder dauerhaft über eine Kunststoffschweiß-Verbindung 18 geschlossen ist. Der Kammerdeckel 17 hat auf der Oberseite einen Anschlussstutzen 19, in den die Durchgangsöffnung 13 mündet.

Die Saugglocke 2, der Verbindungskanal 4, der Ablauf 5 und die Schwimmerventilkammer 11 sind integral mit einem Deckel 20 verbunden, der als Schraubdeckel ausgebildet ist. Hierfür hat der Deckel 20 einen Deckelboden 21 und einen zylindrischen Deckelmantel 22, der am Innenumfang mit einem Innengewinde 23 versehen ist.

Ferner weist die Muttermilchpumpe einen Behälter 24 auf, der am Außenumfang der Behälteröffnung ein Außengewinde 25 aufweist, mit dem der Deckel 20 verschraubt ist.

Der Deckel 20 weist einen Belüftungskanal 26 auf, der einenends in einer inneren Oberfläche an der Innenseite des Deckels 20, die dem Innenraum des Behälters 24 zugewandt ist, und anderenends in einer äußeren Oberfläche des Deckels 20, die der Umgebung zugewandt ist, mündet.

Ferner weist die Muttermilchpumpe 1 einen als Gleichstrommotor mit Schleifern und Kommutator ausgebildeten Elektromotor 27 auf. Der Elektromotor 27 ist über eine Welle 28 mit einer Pumpe 29 gekoppelt, die als Membranpumpe ausgebildet ist. An den Eingang 30 der Pumpe 29 ist eine Saugleitung 31 angeschlossen, in der ein erstes, elektrisch steuerbares Drei/Zwei-Wege-Ventil 32 angeordnet ist. Die Saugleitung 31 ist anderenends über eine Sammelleitung 33 mit dem Anschlussstutzen 19 verbunden.

An den Ausgang 34 der Pumpe 29 ist eine Druckleitung 35 angeschlossen, in der ein zweites, elektrisch betätigbares Drei/Zwei-Wege-Ventil 36 angeordnet ist. Die Druckleitung 35 ist anderenends über die Sammelleitung 33 mit dem Anschlussstutzen 19 verbunden.

An die Sammelleitung 33 ist ein Überdruckventil 37 angeschlossen, das zur Umgebung öffnet.

Die ersten und zweiten Drei/Zwei-Wege-Ventile 32, 36 haben jeweils eine Schaltstellung, in der sie zur Umgebung (d.h. zur Atmosphäre) öffnen.

Ferner umfasst die Muttermilchpumpe 1 eine elektronische Steuerung 38 und eine elektrische Stromversorgung 39. Die elektrische Stromversorgung 39 versorgt die elektronische Steuerung 38, den Elektromotor 27 und die elektrisch schaltbaren ersten und zweiten Drei/Zwei-Wege-Ventile 32, 36 mit Strom.

Die elektronische Steuerung 38 ist mit einem Ein-Aus-Schalter 40 und Mitteln zum Einstellen 41 der Drehzahl des Elektromotors verbunden, die ein Einstellrädchen 42 aufweisen.

Die elektronische Steuerung 38 ist mit dem Elektromotor 27 und den elektrisch betätigbaren ersten und zweiten Drei/Zwei-Wege-Ventilen 32, 36 elektrisch verbunden.

Durch Betätigen des Ein-Aus-Schalters 40 ist die Muttermilchpumpe 1 einschaltbar und ausschaltbar. Der eingeschaltete Zustand der Muttermilchpumpe 1 wird als Betriebszustand und der ausgeschaltete Zustand als Ruhezustand bezeichnet.

Die elektronische Steuerung 38 steuert im Betriebszustand die Drehzahl des Elektromotors 27 entsprechend der mittels der Mittel zum Einstellen 41 eingestellten Pumpendrehzahl.

Ferner steuert die elektronische Steuerung 38 den Elektromotor 27 und die ersten und zweiten Drei/Zwei-Wege-Ventile 32, 36 in regelmäßig wiederkehrenden Zyklen. Jeder Zyklus umfasst zumindest eine Saugphase und eine Druckphase. Gegebenenfalls umfasst der Zyklus auch eine Belüftungsphase.

Die ersten und zweiten Drei/Zwei-Wege-Ventile 32, 36 werden magnetisch gesteuert.

Die Schaltstellung der Drei/Zwei-Wege-Ventile 32, 36 in der Saugphase ist in Fig. 1a dargestellt. Hierbei ist das erste Drei/Zwei-Wege-Ventil 32 bestromt und das zweite Drei/Zwei-Wege-Ventil 36 stromlos.

Das erste Drei/Zwei-Wege-Ventil 32 ist auf Durchgang geschaltet, so dass der Eingang 30 der Pumpe 29 über die Saugleitung 31, die Sammelleitung 33, die Durchgangsöffnung 13 und die Schwimmerventilkammer 11 mit dem Ablauf 5 verbunden ist. Das zweite Drei/Zwei-Wege-Ventil 36 ist mit der Umgebung verbunden, so dass der Ausgang 34 der Pumpe 29 über die Druckleitung 35 und das Ventil 36 mit der Umgebung verbunden ist. Infolgedessen fördert die Pumpe 29 durch die Schwimmerventilkammer 11 hindurch Luft aus der Saugglocke 2 heraus. Aufgrund des Unterdrucks wird Milch abgepumpt, die zum Ablaufventil 6 abströmt und sich dort sammelt. Die abgepumpte Luft wird von der Pumpe 29 in die Umgebung abgegeben.

In der Druckphase ist gemäß Fig. 1b das erste Drei/Zwei-Wege-Ventil 32 stromlos und mit der Umgebung verbunden und das zweite Drei/Zwei-Wege-Ventil 36 bestromt und auf Durchgang geschaltet. Infolgedessen ist der Eingang 30 der Pumpe 29 über die Saugleitung 31 mit der Umgebung verbunden und der Ausgang 34 der Pumpe kommunizierend mit der Saugglocke 2 verbunden. Infolgedessen wird von der Pumpe 29 Luft aus der Umgebung in die Schwimmerventilkammer 11 und damit in den Verbindungskanal 4 und in den Ablauf 5 oberhalb des Entenschnabelventils 6 gefördert. Hierdurch wird dort schnell ein Druckausgleich mit Umgebungsdruck oder sogar ein Überdruck gegenüber dem Umgebungsdruck erreicht. Infolgedessen öffnet das Entenschnabelventil 6 schnell und die angesammelte Milch fließt hinreichend in den Behälter 24 ab.

An die Druckphase kann sich unmittelbar eine weitere Saugphase anschließen. Es ist aber auch möglich, dass vor oder nach der Druckphase eine Belüftungsphase folgt, in der der Verbindungskanal 4 nicht aktiv sondern passiv belüftet wird.

Zum Belüften in einer Belüftungsphase wird lediglich der Elektromotor 27 ausgeschaltet. Bei der Schaltstellung der Ventile in Fig. 1b strömt Luft durch die Saugleitung 31, die Pumpe 29, die Druckleitung 35, die Sammelleitung 33 und die Schwimmerventilkammer 11 in den Verbindungskanal 4 nach bzw. in umgekehrter Richtung hinaus, falls ein Überdruck in der Saugglocke 2 herrscht.

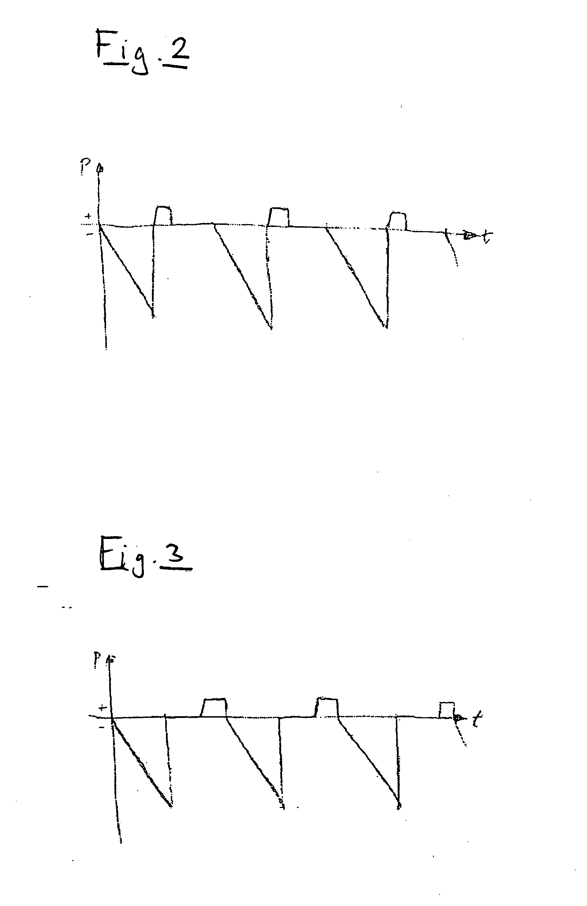

Gemäß Fig. 2 wird in einer Saugphase bei laufendem Elektromotor 27 ein Unterdruck in der Saugglocke 2 erzeugt. Hierbei sind die ersten und zweiten Drei/Zwei-Wege-Ventile 32, 36 so geschaltet, dass die Pumpe 29 Luft aus der Saugglocke 2 abzieht und in die Umgebung abgibt. In der nachfolgenden Druckphase wird bei laufendem Elektromotor 27 aktiv Luft in die Saugglocke 2 gepumpt. Hierfür sind die Drei/Zwei-Wege-Ventile 32, 36 umgeschaltet, so dass die Pumpe 29 Luft aus der Umgebung in die Saugglocke 2 hineinpumpt. Die Druckphase wird durch Abschalten des Elektromotors 27 beendet, wenn der Überdruck hinreichend lange am Ablaufventil 6 anstand, um die Milch in den Behälter 24 abzugeben. In der nachfolgenden Belüftungsphase ist bei unveränderter Ventilstellung die Saugglocke 2 über den Elektromotor 27 mit der Umgebung verbunden, so dass ein Druckausgleich mit der Umgebung stattfindet.

Gemäß Fig. 3 schließt sich an die Saugphase eine Belüftungsphase an. Beim Übergang von der Saugphase in die Belüftungsphase wird die Pumpe 29 abgeschaltet und werden die Drei/Zwei-Wege-Ventile 32, 36 so umgeschaltet, dass Luft über die Pumpe 29 in die Saugglocke 2 nachströmen kann. Hierdurch gleicht sich der Druck in der Saugglocke 2 allmählich an den Umgebungsdruck an. Anschließend wird eine Druckphase durchgeführt, indem lediglich der Elektromotor 27 eingeschaltet wird. Bei unveränderter Ventilstellung wird Luft aus der Umgebung in die Saugglocke 2 hineingefördert, wodurch Milch oder restliche Milch gezwungen wird, durch das Ablaufventil 6 in den Behälter 24 abzuströmen.

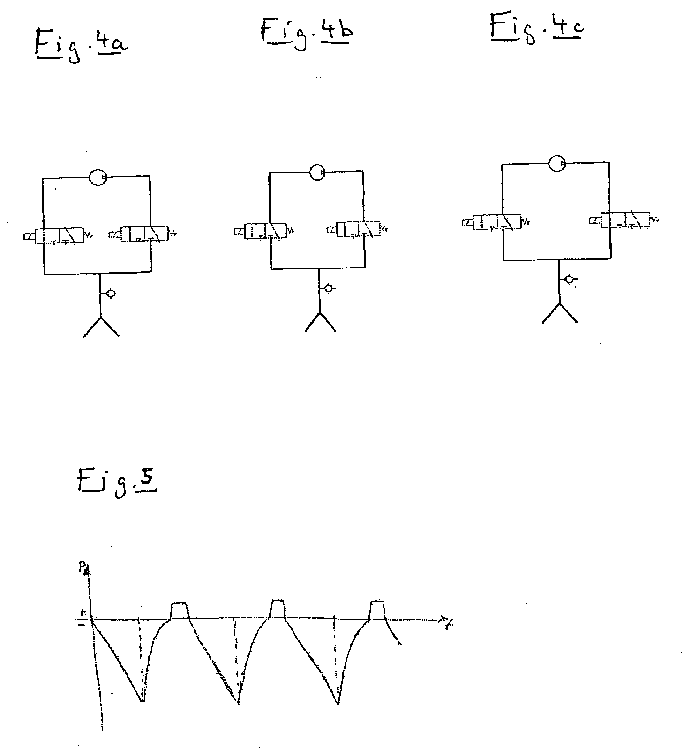

Fig. 4 zeigt eine weitere pneumatische Schaltung, bei der mit einer Belüftungsphase gearbeitet wird. Gemäß Fig. 4a wird in einer Saugphase ein Unterdruck in der Saugglocke 2 erzeugt. Hierfür ist das erste Drei/Zwei-Wege-Ventil 32 auf Durchgang geschaltet, so dass der Eingang 30 der Pumpe 29 kommunizierend mit der Saugglocke 2 verbunden ist. Das zweite Drei/Zwei-Wege-Ventil 36 ist zur Umgebung geöffnet, so dass der Ausgang der Pumpe 29 kommunizierend mit Umgebung verbunden ist.

In einer nachfolgenden Belüftungsphase ist der Elektromotor 27 ausgeschaltet oder kann auch eingeschaltet bleiben. In der Belüftungsphase sind das erste und das zweite Zwei/Drei-Wege-Ventile 32, 36 so geschaltet, dass sie zur Umgebung geöffnet sind. Infolgedessen sind der Eingang 30 und der Ausgang 34 der Pumpe 29 kommunizierend mit Umgebung verbunden. Die Saugleitung 31 und die Druckleitung 35 sind zur Saugglocke 2 hin gesperrt. Aufgrund von Leckagen baut sich dennoch der Druck in der Saugglocke 2 allmählich ab. Je kürzer die Belüftungsphase ist, desto weniger wird der Unterdruck abgebaut. Aufgrund des langsamen Abbaus des Unterdruckes wird die oberhalb des Ablaufventils 6 angesammelte Milch nicht vollständig in den Behälter 24 abgegeben.

Gemäß Fig. 4c wird in einer nachfolgenden Belüftungsphase der Elektromotor 27 eingeschaltet und wird das zweite Drei/Zwei-Wege-Ventil 36 umgeschaltet, so dass der Ausgang der Pumpe 29 kommunizierend mit der Saugglocke 2 verbunden ist. Infolgedessen wird in der Saugglocke 2 ein Überdruck aufgebaut, der bewirkt, dass die Milch durch das Ablaufventil 6 in den Behälter 24 abläuft.

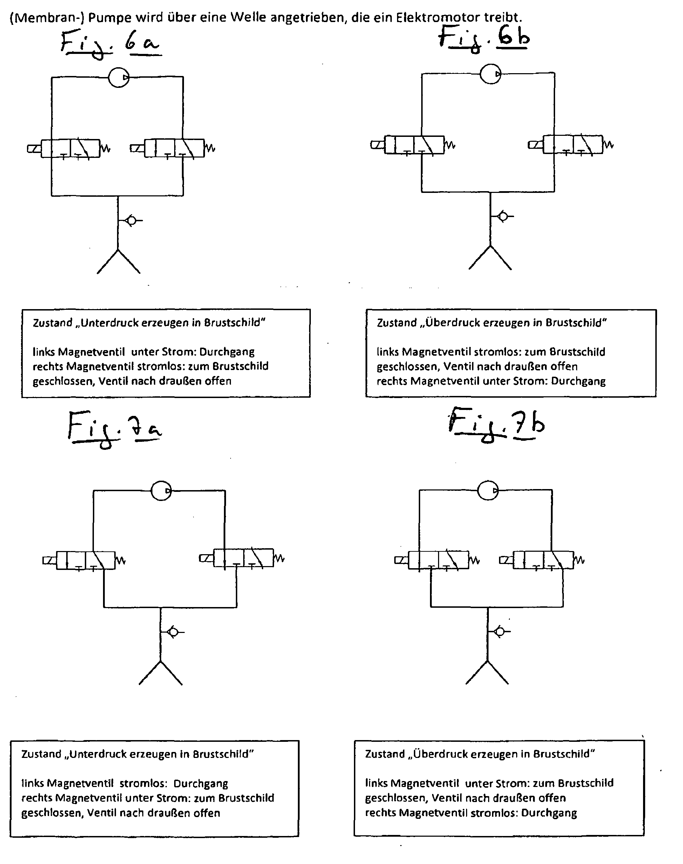

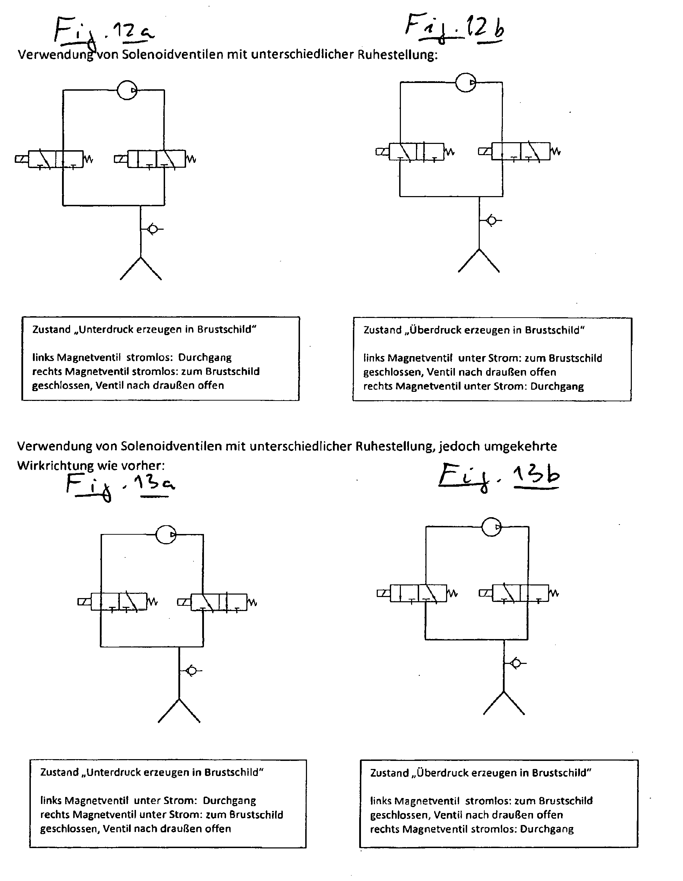

Weitere Varianten der pneumatischen Schaltung sind in den Fig. 6a und b bis 13a und b gezeigt.

Gemäß Fig. 6a ist das erste Drei/Zwei-Wege-Ventil 32 bestromt und auf Durchgang geschaltet. Das zweite Drei/Zwei-Wege-Ventil 36 ist stromlos, so dass es zur Saugglocke 2 hin geschlossen und zur Umgebung hin geöffnet ist. In diesem Zustand wird bei laufendem Elektromotor 27 Unterdruck in der Saugglocke 2 erzeugt.

Gemäß Fig. 6b ist das erste Drei/Zwei-Wege-Ventil 32 stromlos, so dass es zur Umgebung geöffnet und zur Saugglocke 2 hin geschlossen ist. Das zweite Drei/Zwei-Wege-Ventil 36 ist bestromt und auf Durchgang zur Saugglocke 2 geschaltet. In diesem Zustand wird bei laufendem Elektromotor 27 Luft in die Saugglocke 2 hineingepumpt.

Gemäß Fig. 7a ist das erste Drei/Zwei-Wege-Ventil 32 stromlos und auf Durchgang zur Saugglocke 2 geschaltet. Das zweite Drei/Zwei-Wege-Ventil 36 ist bestromt und zur Saugglocke 2 geschlossen und zur Umgebung geöffnet. In diesem Zustand wird bei laufendem Elektromotor 27 ein Unterdruck in der Saugglocke 2 erzeugt.

Gemäß Fig. 7b ist das erste Drei/Zwei-Wege-Ventil 32 bestromt, so dass es zur Saugglocke 2 geschlossen und zur Umgebung geöffnet ist. Das zweite Drei/Zwei-Wege-Ventil 36 ist stromlos, so dass es auf Durchgang zur Saugglocke 2 geschaltet ist. In diesem Zustand wird bei laufendem Elektromotor 27 Luft in die Saugglocke hineingepumpt.

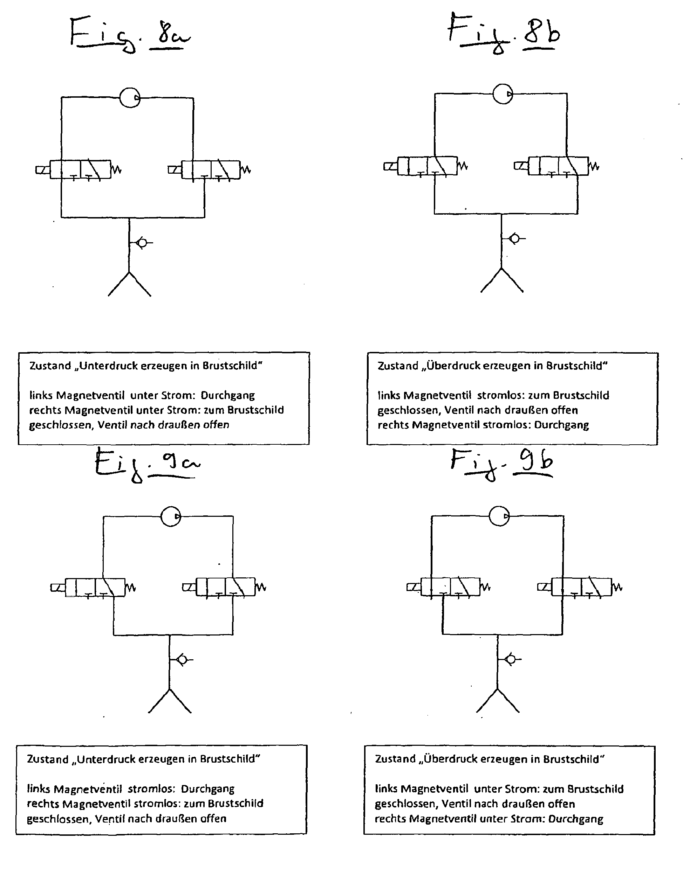

Gemäß Fig. 8a ist das erste Drei/Zwei-Wege-Ventil 32 bestromt und auf Durchgang zur Saugglocke 2 geschaltet. Das zweite Drei/Zwei-Wege-Ventil 36 ist ebenfalls bestromt, so dass es zur Saugglocke 2 hin geschlossen und zur Umgebung hin geöffnet ist. In diesem Zustand wird bei laufendem Elektromotor 27 ein Unterdruck in der Saugglocke 2 erzeugt.

Gemäß Fig. 8b ist das erste Drei/Zwei-Wege-Ventil 32 unbestromt, so dass es zur Saugglocke 2 hin geschlossen und zur Umgebung hin geöffnet ist. Das zweite Drei/Zwei-Wege-Ventil 36 ist ebenfalls stromlos, so dass es auf Durchgang zur Saugglocke 2 geschaltet ist. In diesem Zustand wird bei laufendem Elektromotor 27 Luft in die Saugglocke 2 hineingefördert.

Gemäß Fig. 9a ist das erste Drei/Zwei-Wege-Ventil 32 stromlos und auf Durchgang zur Saugglocke 2 geschaltet. Das zweite Drei/Zwei-Wege-Ventil 36 ist stromlos, so dass es zur Saugglocke 2 hin geschlossen und zur Umgebung hin geöffnet ist. In diesem Zustand wird bei laufendem Elektromotor 27 ein Unterdruck in der Saugglocke 2 erzeugt.

Gemäß Fig. 9b ist das erste Drei/Zwei-Wege-Ventil 32 bestromt, so dass es zur Saugglocke 2 hin geschlossen und zur Umgebung hin geöffnet ist. Das zweite Drei/Zwei-Wege-Ventil 36 ist bestromt, so dass es auf Durchgang zur Saugglocke 2 geschaltet ist. In diesem Zustand wird bei laufendem Elektromotor 27 Luft in die Saugglocke 2 hineingefördert.

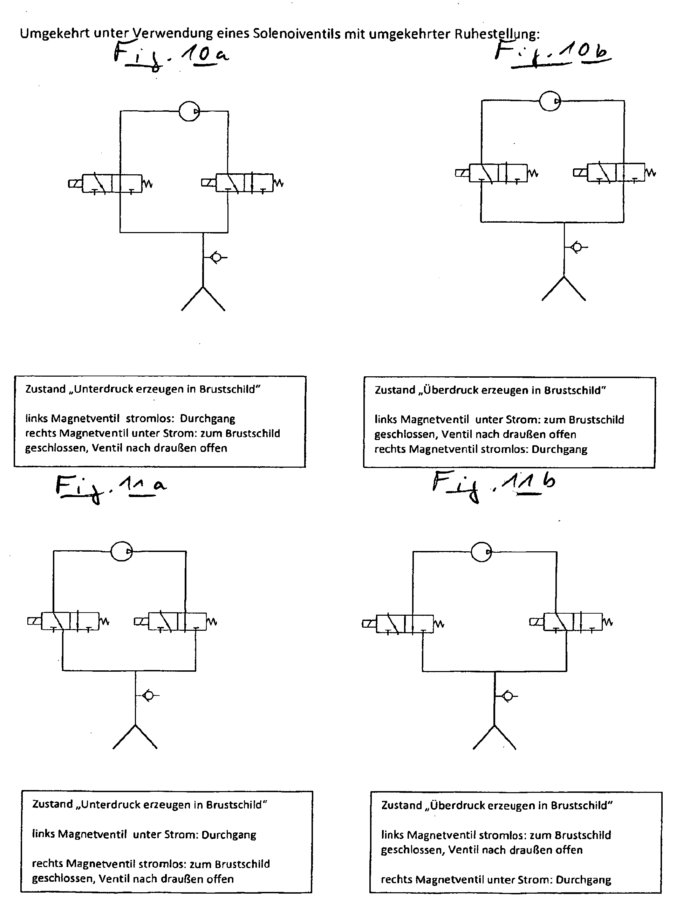

Gemäß Fig. 10a ist das erste Drei/Zwei-Wege-Ventil 32 unbestromt und auf Durchgang zur Saugglocke 2 geschaltet. Das zweite Drei/Zwei-Wege-Ventil 36 ist bestromt, so dass es zur Saugglocke 2 hin geschlossen und zur Umgebung hin geöffnet ist. In diesem Zustand wird bei laufendem Elektromotor 27 ein Unterdruck in der Saugglocke 2 erzeugt.

Gemäß Fig. 10b ist das erste Drei/Zwei-Wege-Ventil 32 bestromt, so dass es zur Saugglocke 2 hin geschlossen ist und zur Umgebung hin geöffnet ist. Das zweite Drei/Zwei-Wege-Ventil 36 ist unbestromt, so dass es auf Durchgang zur Saugglocke 2 geschaltet ist. In diesem Zustand wird bei laufendem Elektromotor 27 Luft in die Saugglocke 2 hineingefördert.

Gemäß Fig. 11a ist das erste Drei/Zwei-Wege-Ventil 32 bestromt und auf Durchgang zur Saugglocke 2 geschaltet. Das zweite Drei/Zwei-Wege-Ventil 36 ist stromlos, so dass es zur Saugglocke 2 hin geschlossen und zum Umgebung hin geöffnet ist. In diesem Zustand wird bei laufendem Elektromotor 27 ein Unterdruck in der Saugglocke 2 erzeugt.

Gemäß Fig. 11b ist das erste Drei/Zwei-Wege-Ventil 32 unbestromt, so dass es zur Saugglocke 2 hin geschlossen und zum Umgebung hin geöffnet ist. Das zweite Drei/Zwei-Wege-Ventil 36 ist bestromt, so dass es auf Durchgang zur Saugglocke 2 geschaltet ist. In diesem Zustand wird bei laufendem Elektromotor 27 Luft in die Saugglocke 2 hineingefördert.

Gemäß Fig. 12a ist das erste Drei/Zwei-Wege-Ventil 32 stromlos, so dass es auf Durchgang zur Saugglocke 2 geschaltet ist. Das zweite Drei/Zwei-Wege-Ventil 36 ist stromlos, so dass es zur Saugglocke 2 geschlossen und zur Umgebung hin geöffnet ist. In diesem Zustand wird bei laufendem Elektromotor 29 Unterdruck in der Saugglocke 2 erzeugt.

Gemäß Fig. 12b ist das erste Drei/Zwei-Wege-Ventil 36 bestromt, so dass es zur Saugglocke 2 hin geschlossen und zur Umgebung hin geöffnet ist. Das zweite Drei/Zwei-Wege-Ventil 36 ist bestromt, so dass es auf Durchgang zur Saugglocke 2 geschaltet ist. In diesem Zustand wird Luft in die Saugglocke 2 hineingefördert.

Gemäß Fig. 13a ist das erste Drei/Zwei-Wege-Ventil 32 bestromt, so dass es auf Durchgang zur Saugglocke 2 geschaltet ist. Das zweite Drei/Zwei-Wege-Ventil 36 ist ebenfalls bestromt, so dass es zur Saugglocke 2 hin geschlossen und zur Umgebung hin geöffnet ist. In diesem Zustand wird bei laufendem Elektromotor 27 ein Unterdruck in der Saugglocke 2 erzeugt.

Gemäß Fig. 13b ist das erste Drei/Zwei-Wege-Ventil 32 stromlos, so dass es zur Saugglocke 2 hin geschlossen und zur Umgebung hin geöffnet ist. Das zweite Drei/Zwei-Wege-Ventil 36 ist stromlos, so dass es auf Durchgang geschaltet ist. In diesem Zustand wird bei laufendem Elektromotor 27 Luft in die Saugglocke 2 hineingefördert.

Die Pneumatikschaltung der Muttermilchpumpe 1 kann unter Berücksichtigung der Dauer der Saugphasen, Druckphasen und gegebenenfalls Belüftungsphasen sowie der Drehzahl des Elektromotors 27 in den Phasen hinsichtlich des minimalen Stromverbrauchs ausgewählt und weiter geändert werden.

## Patentansprüche

1. Elektrische Muttermilchpumpe (1) mit
- einem Elektromotor (27),
- einer von dem Elektromotor (27) angetriebenen Pumpe (29),
- mindestens einer Saugglocke (2),
- einem über einen Verbindungskanal (4) mit der Saugglocke (2) verbundenen Ablauf (5) für Muttermilch,
- einem mit dem Ablauf verbundenen Behälter (24) zum Auffangen von Muttermilch,
- einem im Ablauf (4) angeordneten Ablaufventil (6), das schließt, wenn die Differenz des Druckes im Behälter (24) und in der Saugglocke (2) einen bestimmten Mindestwert aufweist und das öffnet, wenn der Mindestwert unterschritten ist,
- einer Schwimmerventilkammer (11), die unten mit dem Verbindungskanal (4) verbunden ist und oben eine Durchgangsöffnung (13) sowie einen die Durchgangsöffnung (13) umgebenden Dichtsitz (14) hat,
- einem in der Schwimmerventilkammer (11) angeordneten Schwimmerkörper (15), der oben einen Dichtkörper (16) aufweist, der abdichtend am Dichtsitz (14) anliegt, wenn der Schwimmerkörper (15) in der Schwimmerventilkammer (11) aufschwimmt,
- einer die Durchgangsöffnung (13) mit dem Eingang (30) der Pumpe (29) verbindenden Saugleitung (31), **dadurch gekennzeichnet, dass**
- einem in der Saugleitung (31) angeordneten, elektrisch betätigbaren erstem Dreiwegeventil (32),
- einer mit dem Verbindungskanal und dem Ausgang (34) der Pumpe (29) verbundenen Druckleitung (35),
- einem in der Druckleitung (35) angeordneten, elektrisch betätigbaren zweiten Dreiwegeventil (36),
- Mitteln zum Ein- und Ausschalten (40) des Elektromotors (27) und
- einer elektronischen Steuerung (38), die mit den Mitteln zum Ein- und Ausschalten (40), dem Elektromotor (27) und den elektrisch betätigbaren ersten und zweiten Dreiwegeventilen (32, 36) elektrisch verbunden ist und die ausgebildet ist, den Elektromotor (27) in Abhängigkeit von einer Betätigung der Mittel zum Ein- und Ausschalten (40) in einen Betriebszustand oder in einen Ruhezustand zu versetzen, wobei sie im Betriebszustand den Elektromotor (27) und die ersten und zweiten Dreiwegeventile (32, 36) in Zyklen betreibt, die eine Saugphase umfassen, in der der Elektromotor (27) eingeschaltet ist, der Eingang (30) der Pumpe (29) über die Saugleitung (31) und das erste Dreiwegeventil (32) mit der Durchgangsöffnung (14) verbunden ist und der Ausgang der Pumpe (34) über die Druckleitung (35) und das zweite Dreiwegeventil (36) mit der Umgebung verbunden ist, die ferner eine Druckphase umfassen, in denen der Elektromotor (27) eingeschaltet ist, der Eingang (30) der Pumpe (29) über die Saugleitung (31) und das erste Dreiwegeventil (32) mit der Umgebung verbunden ist und der Ausgang (34) der Pumpe (29) über die Druckleitung (35) und das zweite Dreiwegeventil (36) mit dem Verbindungskanal (4) verbunden ist.

2. Muttermilchpumpe nach Anspruch 1, bei der die elektronische Steuerung (38) so ausgebildet ist, dass der Elektromotor (27) in einer Belüftungsphase im Anschluss an die Druckphase oder zwischen der Saugphase und der Druckphase ausgeschaltet ist, der Eingang (30) der Pumpe (29) über die Saugleitung (31) und das erste Dreiwegeventil (32) mit der Umgebung verbunden ist und der Ausgang der Pumpe (29) über die Druckleitung (35) und das zweite Dreiwegeventil (36) mit dem Verbindungskanal (4) verbunden ist.

3. Muttermilchpumpe nach Anspruch 1, bei der die elektronische Steuerung (38) so ausgebildet ist, dass sie in einer Belüftungsphase zwischen der Saugphase und der Druckphase den Eingang (30) der Pumpe (29) über die Saugleitung (31) und das erste Dreiwegeventil (32) und den Ausgang (34) der Pumpe (29) über die Druckleitung (35) und das zweite Dreiwegeventil (36) mit der Umgebung verbindet.

4. Muttermilchpumpe nach einem der Ansprüche 1 bis 3, die Mittel zum Einstellen (41, 42) der Drehzahl des Elektromotors (27) aufweist und bei der die elektronische Steuerung (38) so ausgebildet ist, dass sie den Elektromotor (27) mit einer Drehzahl entsprechend der Einstellung der Mittel zum Einstellen (41, 42) der Drehzahl betreibt.

5. Muttermilchpumpe nach einem der Ansprüche 1 bis 4, bei der die elektronische Steuerung (38) so ausgebildet ist, dass sie entsprechend der Einstellung der Mittel zum Einstellen (41, 42) den Elektromotor (37) kontinuierlich oder in Zyklen betreibt.

6. Muttermilchpumpe nach einem der Ansprüche 1 bis 5, bei der das erste Dreiwegeventil (32) und/oder das zweite Dreiwegeventil (36) ein Drei/Zwei-Wegeventil ist.

7. Muttermilchpumpe nach einem der Ansprüche 1 bis 6, bei der das erste Dreiwegeventil (32) und/oder das zweite Dreiwegeventil (36) ein elektromagnetisches Ventil ist.

8. Muttermilchpumpe nach Anspruch 7, bei der das erste und/oder das zweite Dreiwegeventil (32, 36) ein Solenoid-Ventil ist.

9. Muttermilchpumpe nach einem der Ansprüche 1 bis 8, bei der die Saugglocke (2) über ein Überdruckventil (37) mit der Umgebung verbunden ist.

10. Muttermilchpumpe nach einem der Ansprüche 1 bis 9, bei der der Innenraum des Behälters (24) belüftet ist.

11. Muttermilchpumpe nach einem der Ansprüche 1 bis 10, die einen lösbar mit dem Behälter (24) verbundenen Deckel (20) aufweist, an dem die Saugglocke (2), der Verbindungskanal (4), die Schwimmerventilkammer (11) und der Ablauf (5) mit dem Ablaufventil (6) angeordnet sind.

12. Muttermilchpumpe nach Anspruch 11, bei der der Deckel (20) einen Belüftungskanal (26) aufweist, der sich von einer inneren Oberfläche des Deckels (20) zu einer äußeren Oberfläche des Deckels (20) erstreckt, um den Behälter (24) zu belüften.

## Claims

1. An electrical breast milk pump (1), with
• an electric motor (27),
• a pump (29) driven by the electric motor (27),
• at least one suction cup (2),
• an outlet (5) for breast milk, connected to the suction cup (2) via a connection channel (4),
• a reservoir (24) for collecting breast milk, connected to the outlet,
• an outlet valve (6) disposed in the outlet (4), which closes when the difference between the pressure in the reservoir (24) and that in the suction cup (2) has a certain minimum value, and which opens when the value has fallen below the minimum,
• a floater valve chamber (11) which is connected to the connection channel (4) at the downside, and has a passage opening (13) at the topside, as well as a seal seat (14) surrounding the passage opening (13),
• a floater body (15), disposed in the floater valve chamber (11), which has a sealing body (16) at the topside which bears sealingly against the seal seat (14) when the floater body (15) floats up in the floater valve chamber (11),
• a suction line (31), connecting the passage opening (13) to the inlet (30) of the pump (29), **characterized in that**
• an electrically activated first three-way valve (32), disposed in the suction line (31),
• a pressure line (35), connected to the connection channel and the outlet (34) of the pump (29),
• an electrically activated second three-way valve (36), disposed in the pressure line (35)
• means (40) for switching the electric motor (27) on and off, and
• an electronic control system (38), which is electrically connected to the means (40) for switching on and off, the electric motor (27) and the electrically activated first and second three-way valves (32, 36), and which is configured to set the electric motor (27) into an operating mode or an idle mode depending on an activation of the means (40) for switching on and off, wherein in the operating mode, it operates the electric motor (27) and the electrically activated first and second three-way valves (32, 36) in cycles, which comprise a suction phase, in which the electric motor (27) is switched on, the inlet (30) of the pump (29) is connected to the passage opening (14) via the suction line (31) and the first three-way valve (32) and the outlet (34) of the pump (29) is connected to the surroundings via the pressure line (35) and the second three-way valve (36), and which further comprise a pressure phase, in which the electric motor (27) is switched on, the inlet (30) of the pump (29) is connected to the surroundings via the suction line (31) and the first three-way valve (32) and the outlet (34) of the pump (29) is connected to the connection channel (4) via the pressure line (35) and the second three-way valve (36).

2. The breast milk pump according to claim 1, wherein the electronic control system (38) is configured such that the electric motor (27) is switched off in a venting phase subsequent to the pressure phase or between the suction phase and the pressure phase, the inlet (30) of the pump (29) is connected to the surroundings via the suction line (31) and the first three-way valve (32), and the outlet of the pump (29) is connected to the connection channel (4) via the pressure line (35) and the second three-way valve (36).

3. The breast milk pump according to claim 1, wherein the electronic control system (38) is configured such that in a venting phase between the suction phase and the pressure phase, it connects the inlet (30) of the pump (29) via the suction line (31) and the first three-way valve (32), and the outlet (34) of the pump (29) via the pressure line (35) and the second three-way valve (36) to the surroundings.

4. A breast milk pump according to any one of claims 1 to 3, which has means (41, 42) for adjusting the rotational speed of the electric motor (27) and wherein the electronic control system (38) is configured such that it operates the electric motor (27) with a speed according to the setting of the means (41, 42) for adjusting the rotational speed.

5. A breast milk pump according to any one of claims 1 to 4, wherein the electronic control system (38) is configured such that it operates the electric motor (27) continuously or in cycles according to the setting of the means (41, 42) for adjusting.

6. A breast milk pump according to any one of claims 1 to 5, wherein the first three-way valve (32) and/or the second three-way valve (36) is a three/two-way valve.

7. A breast milk pump according to any one of claims 1 to 6, wherein the first three-way valve (32) and/or the second three-way valve (36) is an electromagnetic valve.

8. The breast milk pump according to claim 7, wherein the first and/or the second three-way valve (32, 36) is a solenoid valve.

9. A breast milk pump according to any one of claims 1 to 8, wherein the suction cup (2) is connected to the surroundings via a pressure relief valve (37).

10. A breast milk pump according to any one of claims 1 to 9, wherein the interior of the reservoir (24) is vented.

11. A breast milk pump according to any one of claims 1 to 10, which has a lid (20) detachably connected to the reservoir (24), on which the suction cup (2), the connection channel (4), the floater valve chamber (11) and the outlet (5) with the outlet valve (6) are disposed.

12. The breast milk pump according to claim 11, wherein the lid (20) has a venting channel (26) which extends from an inner surface of the lid (20) to an outer surface of the lid (20) in order to vent the reservoir (24).

## Revendications

1. Tire-lait électrique (1) avec
• un moteur électrique (27),
• une pompe (29) entraînée par le moteur électrique (27),
• au moins une cloche aspirante (2),
• un déversoir (5) pour lait maternel, relié à la cloche d'aspiration (2) à travers un canal de liaison (4)
• un récipient (24), relié au déversoir, pour recueillir du lait maternel
• une soupape de vidange (6) arrangée dans le déversoir (4), qui se ferme quand la différence entre la pression dans le récipient (24) et dans la cloche d'aspiration (2) a une certaine valeur minimale et qui s'ouvre quand la valeur minimale n'est pas atteinte,
• une chambre de soupape à flotteur (11), qui est reliée avec le canal de liaison (4) en bas et a une ouverture de passage (13) et un siège d'étanchéité (14) entourant l'ouverture de passage (13) sur le haut,
• un corps flotteur (15) arrangé dans la chambre de soupape à flotteur (11), qui a un corps d'étanchéité (16) en haut qui reste sur le siège d'étanchéité (14) de manière étanche quand le corps flotteur (15) flotte dans la chambre de soupape à flotteur (11),
• un conduit d'aspiration (31) reliant l'ouverture de passage (13) à l'entrée (30) de la pompe (29),
**caractérisé par**
• une première soupape à trois voies (32) arrangée dans le conduit d'aspiration (31) et qui peut être mise en action de manière électrique,
• un conduit de pression (35) relié au canal de liaison et à la sortie (34) de la pompe (29),
• une deuxième soupape à trois voies (36) arrangée dans le conduit de pression (35) et qui peut être mise en action de manière électrique,
• des moyens pour mettre en marche et hors marche (40) le moteur électrique (27), et
• une commande électronique (38) qui est électriquement reliée aux moyens (40) pour mettre en marche et hors marche, au moteur électrique (27) et à les soupapes à trois voies première et deuxième qui peuvent mises en action de manière électrique, et qui est construite de mettre le moteur électrique (27) dans un état de fonctionnement ou de repos en fonction d'un actionnement des moyens (40) pour mettre en marche et hors marche, dans l'état de fonctionnement la commande (38) opérant le moteur électrique (27) et les soupapes à trois voies première et deuxième (32, 36) en des cycles qui comportent une phase d'aspiration, dans laquelle le moteur électrique (27) est mis en marche, l'entrée (30) de la pompe (29) est reliée à l'ouverture de passage (14) à travers le conduit d'aspiration (31) et la première soupape à trois voies (32), et la sortie (34) de la pompe est reliée aux environs à travers le conduit de pression (35) et la deuxième soupape à trois voies (36), qui comportent en outre une phase de pression dans laquelle le moteur électrique (27) est mis en marche, l'entrée (30) de la pompe (29) est reliée aux environs à travers le conduit d'aspiration (31) et la première soupape à trois voies (32), et la sortie (34) de la pompe est reliée au canal de liaison (4) à travers le conduit de pression (35) et la deuxième soupape à trois voies (36).

2. Tire-lait selon la revendication 1, dans lequel la commande électronique (38) est construite de telle manière que dans une phase d'aération suite à la phase de pression ou entre la phase d'aspiration et la phase de pression, le moteur électrique (27) est mis hors de marche, l'entrée (30) de la pompe (29) est reliée aux environs à travers le conduit d'aspiration (31) et la première soupape à trois voies (32), et la sortie de la pompe (29) est reliée au canal de liaison (4) à travers le conduit de pression (35) et la deuxième soupape à trois voies (36).

3. Tire-lait selon la revendication 1, dans lequel la commande électronique (38) est construite de telle manière que dans une phase d'aération entre la phase d'aspiration et la phase de pression, elle relie l'entrée (30) de la pompe (29) à travers le conduit d'aspiration (31) et la première soupape à trois voies (32) aux environs, et aussi aux environs la sortie (34) de la pompe (29) à travers le conduit de pression (35) et la deuxième soupape à trois voies (36).

4. Tire-lait selon l'une quelconque des revendications 1 à 3, qui a des moyens pour ajuster (41, 42) la vitesse de rotation du moteur électrique (27) et dans lequel la commande électronique (38) est construite de telle manière qu'elle opère le moteur électrique (27) avec une vitesse de rotation correspondante à l'ajustage des moyens pour ajuster (41, 42) la vitesse de rotation.

5. Tire-lait selon l'une quelconque des revendications 1 à 4, dans lequel la commande électronique (38) est construite de telle manière qu'elle opère le moteur électrique (27) continument ou en des cycles, conforme à l'ajustage des moyens pour ajuster (41, 42).

6. Tire-lait selon l'une quelconque des revendications 1 à 5, dans lequel la première soupape à trois voies (32) et/ou la deuxième soupape à trois voies (36) est une soupape à trois/deux voies.

7. Tire-lait selon l'une quelconque des revendications 1 à 6, dans lequel la première soupape à trois voies (32) et/ou la deuxième soupape à trois voies (36) est une soupape électromagnétique.

8. Tire-lait selon la revendication 7, dans lequel la première soupape à trois voies (32) et/ou la deuxième soupape à trois voies (36) est une soupape à solénoïde.

9. Tire-lait selon l'une quelconque des revendications 1 à 8, dans lequel la cloche aspirante (2) est reliée aux environs à travers une soupape de surpression (37).

10. Tire-lait selon l'une quelconque des revendications 1 à 9, dans lequel l'espace intérieur du récipient (24) est aéré.

11. Tire-lait selon l'une quelconque des revendications 1 à 10, qui a un couvercle (20) relié au récipient (24) de manière détachable et sur lequel sont arrangés la cloche aspirante (2), .le canal de liaison (4), la chambre de soupape à flotteur (11) et le déversoir (5) avec la soupape de vidange (6).

12. Tire-lait selon la revendication 12, dans lequel le couvercle (20) comporte un canal d'aération (26) qui s'étend de la surface intérieure du couvercle (20) jusqu'à une surface extérieure du couvercle (20) afin d'aérer le récipient (24).
